(19) Europäisches Patentamt
European Patent Office
Office européen des brevets

(11) Publication number: 0 175 005 A1

(12) EUROPEAN PATENT APPLICATION

(21) Application number: 84110645.3

(22) Date of filing: 06.09.84

(51) Int. Cl.⁴: C 07 D 215/20, A 61 K 31/47, C 07 D 215/14

(43) Date of publication of application: 26.03.86 Bulletin 86/13

(84) Designated Contracting States: AT BE CH DE FR GB IT LI LU NL SE

(71) Applicant: USV PHARMACEUTICAL CORPORATION, 1 Scarsdale Road, Tuckahoe New York (US)

(72) Inventor: Musser, John H., 1009 Millstream Road, Malvern Pennsylvania (US)
Inventor: Sutherland, Charles A., 222 Elwood Avenue, Hawthorne New York (US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)

(54) **5,6,7-Quinoline carbamates.**

(57) Compounds of the structure:

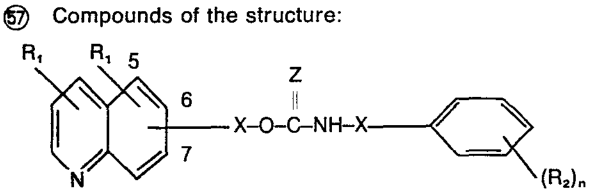

and salts thereof, wherein:

each $R_1$ is H, alkyl, alkoxy, carboxyl, alkylcarboxy, acylamino, dialkylamino, trihalomethyl, halogen, nitro, hydroxy or cyano;

each $R_2$ is H, monosubstituted or independdently disubstituted hydroxy, alkyl, halogen, nitro, alkoxy, carboxyl, alkylcarboxy, trihalomethyl, cyano, dialkylamino or acylamino;

Z is O ir S;

each X is an alkylene chain containing up to 7 carbon atoms in the principal chain and a total of up to 10 carbon atoms; and

n is 1 or 2.

EP 0 175 005 A1

# 5,6,7-QUINOLINE CARBAMATES

It has now been found that quinoline carbamates are active antilipolytic agents as evidenced by the myocardial lipase and the rat adipocyte assays.

Lipolysis is associated with ischemic heart disease: free fatty acid has a detrimental effect on the ischemic heart by disrupting electrical conduction, decreasing myocardial efficiency and preventing the transfer of adenosine diphosphate and adenosine triphosphate, in and out, respectively, of the mitochondria. Interventions which depress myocardial oxygen consumption in animals and man provide a protective effect against ischemic injury.

This invention relates to compounds useful in the treatment of ischemic heart disease and hypertriglyceridemia and having the structure:

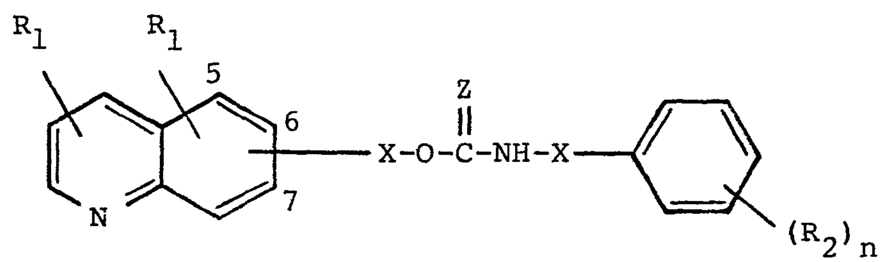

and salts thereof, wherein:

each $R_1$ is H, alkyl, alkoxy, carboxyl, alkylcarboxy, acylamino, dialkylamino, trihalomethyl, halogen, nitro, hydroxy or cyano;

each $R_2$ is H, monosubstituted or independently disubstituted hydroxy, alkyl, halogen, nitro, alkoxy, carboxyl, alkylcarboxy, trihalomethyl, cyano, dialkylamino or acylamino;

Z is O or S;

each X is an alkylene chain containing up to 7 carbon atoms in the principal chain and a total of up to 10 carbon atoms; and

n is 1 or 2.

The alkyl group and the alkyl moieties in alkoxy and alkylcarboxy contain 1 to 7 carbon atoms and may be a straight or a branched chain. Such groups include methyl, ethyl, propyl, isopropyl, butyl, isobutyl, amyl, isoamyl and the like.

The trihalo and halogen include F, Cl, Br or I.

The aryl group is preferably phenyl or naphthyl.

The compounds of this invention may be readily prepared by reacting the appropriate quinolinol or quinolylalkanol with various substituted phenyl or phenylalkyl isocyanates. The reaction is thermally reversible and for that reason the preferred procedure involves the mixing of the two reactants in ethyl ether with a small amount of triethylamine present as a catalyst and stirring at room temperature for several days.

The procedure for preparing compounds in which Z is O is as follows:

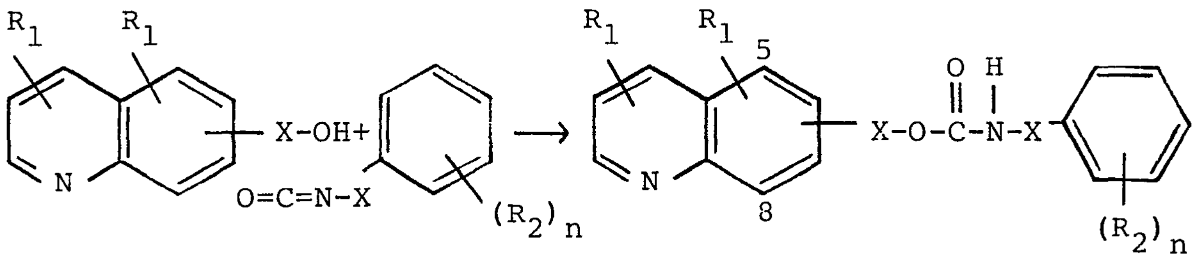

III II

For compounds in which Z is S, the corresponding isothiocyanates are used as reactants. The procedure is accomplished by merely contacting the reactants under carbamate forming conditions, i.e., in a solvent allowing the reaction to proceed at ambient, i.e., room, temperature. The use of higher temperatures up to the reflux temperature of the reaction mixture will shorten the time required. A wide variety of solvents can be employed, the sole requirement being that the solvent be substantially unreactive with the reactants and capable of dissolving the reactants. Diethyl ether, dioxane, tetrahydrofuran and similar solvents can be employed. After the reaction is complete, the product can be isolated by any of the commonly employed procedures, e.g., allowing the product to crystallize from the reaction mixture on cooling, with or without concentration of the reaction mixture before cooling by removal of solvent.

Salts of the present new compounds can be formed using acids such as hydrochloric, perchloric, sulfuric, nitric, benezesulfonic and toluene sulfonic acids as well as other acids commonly employed for

this purpose. Those compounds which contain a free carboxy group can form salts with bases such as sodium hydroxide, potassium hydroxide, potassium carbonate, sodium bicarbonate, calcium hydroxide and the like. Of particular value are the salts which are pharmaceutically acceptable.

The desired starting materials and intermediates can be prepared from readily available material using standard organic reactions.

The invention will be more fully illustrated in the examples that follow. These examples are given by way of illustration and are not to be considered as limiting.

EXAMPLE 1

To a solution of 7-hydroxyquinoline (1.45 g., Pfaltz and Bauer H17880) and 4-methoxyphenyl isocyanate (1.49 g.) in ethyl ether (100 ml) was added triethylamine (1.4 ml). The reaction was stirred for two days at room temperature. The resulting precipitate was filtered and recrystallized from ethyl ether.

In like manner as above, using appropriate starting materials and reagents, the following compounds were prepared:

EXAMPLE 2

6-Quinolyl-4-methoxy carbanilate.

EXAMPLE 3

5-Quinolyl-4-methoxy carbanilate.

EXAMPLE 4

7-Quinolyl-2-methoxy carbanilate.

EXAMPLE 5

6-Quinolyl-2-methoxy carbanilate.

EXAMPLE 6

5-Quinolyl-2-methoxy carbanilate.

EXAMPLE 7

7-Quinolyl-3-methoxy carbanilate.

EXAMPLE 8

7-Quinolyl-4-carbethoxy carbanilate.

EXAMPLE 9

7-Quinolyl-2-chloro carbanilate.

EXAMPLE 10

7-Quinolyl-2-methyl carbanilate.

EXAMPLE 11

7-Quinolyl-2-trifluoromethyl carbanilate.

EXAMPLE 12

7-Quinolyl-4-methoxy phenethyl carbamate.

The compounds of the present invention exhibit activity in the myocardial lipase assay and the rat adiopocyte assay.

Myocardial Lipase Assay

All compounds were dissolved in DMSO (final concentration 3.0%) and tested in duplicate at a concentration of 100 µM. Canine cardiac lipases were obtained by extracting washed heart membranes with buffer plus heparin and a small amount of triton X-100 detergent. Because these enzymes are only active at an oil-water interface, the enzyme reaction is run in an oil water-elusion that contains triolein substrate, tris buffer (50 mM, ph 6.8) and a small amount of bovine serum albumin (0.5%) added to stabilize the emulsion. A small amount of tritatiated triolein was added to the unlabelled triolein substrate. Tritium-labelled oleic acid released by the lipases was extracted into hexane, separated from unreacted triolein and counted in a scintillation counter. Inhibitory agents reduce the amount of radioactivity appearing in the free fatty acid fraction isolated in the extraction procedure.

Rat Adipocyte Assay

Abdonimal fat pads were removed from male rats weighing 200-250 grams and placed in Krebs-bicarbonate buffer gassed with 95% $O_2$/5% $CO_2$. The fat pads were digested with collagenase for 1 hour at 37°C., washed twice with Krebs-bicarbonate buffer and distributed among a set of 20 ml plastic counting vials. Two such vials received only buffer and cells (4 ml) but no agonists or antagonists. The remaining

vials received epinephrine (3 μM) plus the phosphodiesterase inhibitor methylisobutylxanthine (10 μM). Test compounds were dissolved in DMSO or water to a concentration of 20 mM and 40 μl was added to the buffer plus cells in the counting vials. The final compound concentration for routine screening was 200 μM (final concentration of DMSO=1%).

The cells were incubated for 1 hour at 37°C. under 95% $O_2$/5% $CO_2$ atmosphere. The incubation was stopped by placing the vials in crushed ice. The cells and medium were transferred to test tubes, centrifuged and the cell layer removed by aspiration. The aqueous phase was assayed for glycerol using the enzyme glycerol dehydrogenase.

The glycerol dehydrogenase assay for glycerol depends on the enzyme catalyzed conversion of glycerol to glyceraldehyde and NAD to NADH. The assay can detect as little as 5-10 nanomoles of glycerol. The aqueous phase, following removal of cells, usually contained about 50 to 80 nanomoles of glycerol per 300 μl of assayed sample if no inhibitory activity was present. Samples from the control tubes (no agonist) usually contained 0-5 nanomoles of glycerol per 300 μl of sample.

The results obtained on representative compounds of the present invention are shown in Table I.

The therapeutic compounds of this invention may be administered to a mammal alone or in combination with pharmaceutically acceptable carriers, the proportion of which is determined by the solubility and chemical nature of the compound, chosen route of administration and standard pharmaceutical practice.

The physician will determine the dosage of the present therapeutic agents which will be most suitable and it will vary with the form of administration and the particular compound chosen, and also, it will vary with the particular patient under treatment. He will generally wish to initiate treatment with small dosages substantially less than the optimum dose of the compound and increase the dosage by small increments until the optimum effect under the circumstances is reached. The therapeutic dosage will generally be from a couple of mg. to about 30 mg/kg of body weight or higher.

Salts of the present new compounds can be prepared by using standard known procedures by reacting the selected acid or base with the new compound. Salts with acids such as hydrochloric, nitric, sulfuric, benzenesulfonic, toluenesulfonic and similar acids are particularly useful for therapeutic purposes. Salts of the those compounds including free carboxyl groups can be formed with bases to provide the sodium, potassium and calcium salts which are also useful for therapeutic purposes.

TABLE I INHIBITION OF MYOCARDIAL LIPASE AND RAT ADIPOCYTE LIPOLYSIS BY QUINOLINE CARBAMILATES

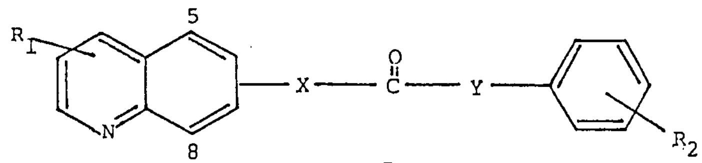

| No. | X | Y | $R_2$ | $R_1$ | M.P. (°C) | Lipase $I_{50}$ µM or % Inh. at 100 µM | Adipocyte $I_{50}$ µM or % Inhib. at µM |
|---|---|---|---|---|---|---|---|
| 1 | 7-0 | NH | 4-$OCH_3$ | H | 153-159 | 60 % | 0% at 30 µM |
| 2 | 6-0 | NH | 4-$OCH_3$ | H | 139-141 | 64 % | 8% at 30 µM |
| 3 | 5-0 | NH | 4-$OCH_3$ | H | 174-177 | 63 % | 1% at 30 µM |
| 4 | 7-0 | NH | 2-$OCH_3$ | H | 115-117 | 48 µM | 80 µM |
| 5 | 6-0 | NH | 2-$OCH_3$ | H | 112-113 | 42 µM | 30 µM |
| 6 | 5-0 | NH | 2-$OCH_3$ | H | 117-118 | 48 µM | 31% at 200 µM |
| 7 | 7-0 | NH | 3-$OCH_3$ | H | 136-137 | 52 % | 25% at 200 µM |
| 8 | 7-0 | NH | 4-$CO_2Et$ | H | 180-182 | 46 % | 18% at 200 µM |
| 9 | 7-0 | NH | 2-Cl | H | 152-154 | 6 % | -- |
| 10 | 7-0 | NH | 2-$CH_3$ | H | 145-147 | 45 % | 20% at 200 µM |
| 11 | 7-0 | NH | 2-$CF_3$ | H | 235-238 | 12 % | -- |
| 12 | 7-0 | $\overset{H}{N}CH_2CH_2$ | 4-$OCH_3$ | H | 112-115 | 97 % | 84% at 100 µM |

WHAT IS CLAIMED IS:

1. A compound of the structure:

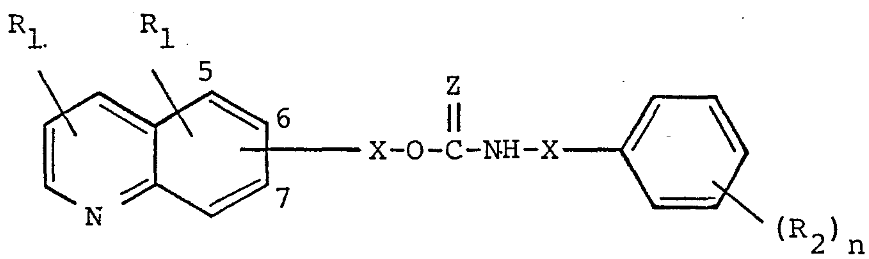

and salts thereof, wherein:

each $R_1$ is H, alkyl, alkoxy, carboxyl, alkylcarboxy, acylamino, dialkylamino, trihalomethyl, halogen, nitro, hydroxy or cyano;

each $R_2$ is H, monosubstituted or independently disubstituted hydroxy, alkyl, halogen, nitro, alkoxy, carboxyl, alkylcarboxy, trihalomethyl, cyano, dialkylamino or acylamino;

Z is O or S;

each X is an alkylene chain containing up to 7 carbon atoms in the principal chain and a total of up to 10 carbon atoms; and

n is 1 or 2.

2. The compound of Claim 1 wherein the alkyl group and the alkyl moieties in alkoxy are straight chain.

3. The compound of Claim 1 wherein the alkyl group and the alkyl moieties in alkoxy and alkylcarboxy are branched chains.

4. The compound of Claim 1 wherein the alkyl group and the alkyl moieties in alkoxy and alkylcarboxy contain from 1 to 7 carbon atoms.

5. The compound of Claim 1 wherein the alkyl group and the alkyl moieties in alkoxy and alkylcarboxy are methyl, ethyl, propyl, isopropyl, butyl, isobutyl, amyl or isoamyl.

6. The compound of Claim 1 wherein the halogen and the halogen moiety in trihalomethyl is F, Cl, Br or I.

7. The compound of Claim 1 wherein the aryl group is phenyl or naphthyl.

8. 7-Quinolyl-4-methoxy phenethyl carbamate;
6-Quinolyl-4-methoxy carbanilate;
5-Quinolyl-2-methoxy carbanilate;
7-Quinolyl-3-methoxy carbanilate;
7-Quinolyl-2-methyl carbanilate; and
7-Quinolyl-4-carbethoxy carbanilate.

9. A therapeutic composition comprising a compound as in any of Claims 1-8 or a pharmaceutically acceptable salt thereof and a pharmaceutical extending agent.

Subsequently filed

CLAIMS FOR AUSTRIA:

1. A process for preparing compounds of the general formula I

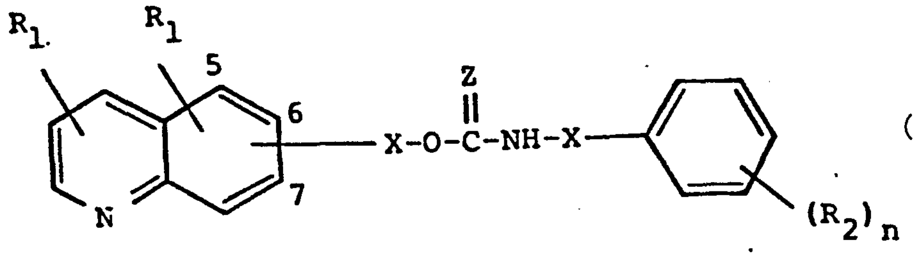

(I)

and salts thereof, wherein:

each $R_1$ is H, alkyl, alkoxy, carboxyl, alkylcarboxy, acylamino, dialkylamino, trihalomethyl, halogen, nitro, hydroxy or cyano;

each $R_2$ is H, monosubstituted or independently disubstituted hydroxy, alkyl, halogen, nitro, alkoxy, carboxyl, alkylcarboxy, trihalomethyl, cyano, dialkylamino or acylamino;

Z is O or S;

each X is an alkylene chain containing up to 7 carbon atoms in the principal chain and a total of up to 10 carbon atoms; and

n is 1 or 2.

which comprises contacting under carbamate forming conditions a compound of formula II

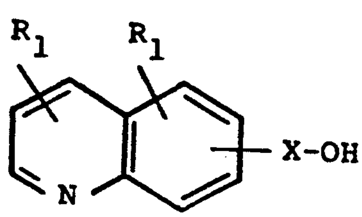

(II)

with a compound of formula III

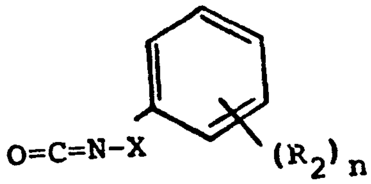

(III)

or the corresponding isothiocyanates and optionally forming salts with bases.

2. The process according to claim 1, characterized in that the alkyl group and the alkyl moieties in alkoxy are straight chain.

3. The process according to claim 1, characterized in that the alkyl group and the alkyl moieties in alkoxy and alkylcarboxy are branched chains.

4. The process according to claim 1, characterized in that the alkyl group and the alkyl moieties in alkoxy and alkylcarboxy contain from 1 to 7 carbon atoms.

5. The process according to claim 1, characterized in that the alkyl group and the alkyl moieties in alkoxy and alkylcarboxy are methyl, ethyl, propyl, isopropyl, butyl, isobutyl, amyl or isoamyl.

6. The process according to claim 1, characterized in that the halogen and the halogen moiety in trihalomethyl is F, Cl, Br or I.

7. The process according to claim 1, characterized in that the aryl group is phenyl or naphthyl.

8. The process according to claim 1, characterized in that the product is
7-Quinolyl-4-methoxy phenethyl carbamate;
6-Quinolyl-4-methoxy carbanilate;
5-Quinolyl-2-methoxy carbanilate;

7-Quinolyl-3-methoxy carbanilate;
7-Quinolyl-2-methyl carbanilate; or
7-Quinolyl-4-carbethoxy carbanilate.

9. A process for preparing a therapeutic composition comprising formulating a compound according to any of claims 1 to 8 or a pharmaceutically acceptable salt thereof with usual carriers and/or diluents.

European Patent Office

# EUROPEAN SEARCH REPORT

0175005

Application number

EP 84 11 0645

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | EP-A-0 116 938 (USV)<br>* Claim 1; page 12, lines 23-25; pages 13-18 *<br>--- | 1,9 | C 07 D 215/20<br>A 61 K 31/47<br>C 07 D 215/14 |
| A | DE-A-3 042 785 (HOECHST)<br>* Claims 1,3; pages 9,10; page 11, lines 1-22 *<br>--- | 1,9 | |
| A | US-A-2 749 347 (CHEMO)<br>* Claim 1 *<br>--- | 1,9 | |
| A | GB-A-1 118 300 (BRISTOL-MYERS)<br>* Claims 1,26 *<br>----- | 1,9 | |
| | | | TECHNICAL FIELDS SEARCHED (Int. Cl.4) |
| | | | C 07 D 215/00<br>A 61 K 31/00 |

The present search report has been drawn up for all claims

| Place of search | Date of completion of the search | Examiner |
|---|---|---|
| THE HAGUE | 23-04-1985 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document